# EUROPEAN PATENT SPECIFICATION

(11) **EP 2 942 000 B1**
(45) Date of publication and mention of the grant of the patent: **06.03.2019**
(21) Application number: 13870308.7
(22) Date of filing: 11.09.2013
(51) Int. Cl.: A61B 1/00, G02B 23/24

(54) **ENDOSCOPE FOGGING PREVENTION SYSTEM**
ENDOSKOP-BESCHLAGSCHUTZSYSTEM
SYSTÈME DE PRÉVENTION DE LA FORMATION DE BUÉE SUR UN ENDOSCOPE

(30) Priority: 04.01.2013 JP 2013000141
(43) Date of publication of application: 11.11.2015
(73) Proprietor: Olympus Corporation, Tokyo 192-8507 (JP)
(72) Inventor: IDE, Takayuki, Tokyo 151-0072 (JP); SUGIYAMA, Yuta, Tokyo 151-0072 (JP); YASUNAGA, Shinji, Tokyo 151-0072 (JP)
(74) Representative: Winter, Brandl, Fürniss, Hübner, Röss, Kaiser, Polte - Partnerschaft mbB
(86) International application number: PCT/JP2013/074595
(87) International publication number: WO 2014/106913

(56) References cited:
- EP-A2- 1 803 388
- WO-A1-2012/039398
- WO-A1-2012/039398
- JP-A- 2008 259 611
- US-A1- 2008 076 087

## Description

### Technical Field

The present invention relates to an endoscope fogging prevention system and an endoscope fogging prevention method for prevent fogging which occurs in an endoscope.

### Background Art

The inside of a body cavity into which an insertion portion of an endoscope is inserted is under an environment, for example, of a temperature of about 35°C to about 37°C and a humidity of about 98% to about 100%. The inside of a distal end portion of the insertion portion is generally lower in temperature than the inside of the body cavity. Thus, when the distal end portion of the insertion portion is inserted in the body, fogs occurs in a lens cover (an observation window) provided at the distal end portion due to, for example, the temperature difference between the endoscope and the inside of the body. This fog may block a field of view during observation.

Thus, recently, the lens cover is heated by a heater to prevent the fog. The heater is controlled based on the temperature of the lens cover which a temperature sensor detected.

An endoscope having such a fogging prevention function therein has been disclosed in, for example, Patent Literature 1. In Patent Literature 1, heating is forced to stop when a heater or a temperature sensor has broken down.

### Citation List

### Patent Literature

Patent Literature 1: International Publication No. 2012/039398

### Summary of Invention

### Technical Problem

However, in Patent Literature 1, when the judgment accuracy of a breakdown is not high, the heater or the temperature sensor may be misjudged to have broken down even if it has not broken down in conditions of use or in a state of use. If the range of allowable tolerance is wider for the prevention of wrong operation, a breakdown that is originally judged as a breakdown may not be judged as a breakdown.

The present invention has been made under these circumstances, and is intended to provide an endoscope fogging prevention system and an endoscope fogging prevention method that capable to high accurately judge a breakdown.

### Solution to Problem

An aspect of an endoscope fogging prevention system of the present invention comprises the features of claim 1. The endoscope fogging prevention system includes a heating section which is provided inside a distal end portion of an endoscope insertion portion and which heats the inside of the distal end portion to prevent the fogging which occurs in an optical
member provided inside the distal end portion, a temperature measurement section which measures heat quantity information regarding the inside of the distal end portion and a control unit which controls the driving of the heating section based on the heat quantity information regarding the inside of the distal end portion measured by the temperature measurement section, wherein the endoscope fogging prevention system judging the operating characteristics of the heating section and the operating characteristics of the temperature measurement section based on the heat quantity information regarding the inside of the distal end portion, and deactivating the heating section when judging that the judgment result is out of a desired range.

### Advantageous Effects of Invention

According to the present invention, it is possible to provide an endoscope fogging prevention system and an endoscope fogging prevention method that capable to high accurately judge a breakdown.

### Brief Description of Drawings

FIG. 1 is a diagram showing the internal structure of a distal end portion of an insertion portion of an endoscope according to the present invention;
FIG. 2 is a diagram showing the structures of a heater and a temperature sensor;
FIG. 3 is a diagram showing Configurations 1 and 2 of an endoscope fogging prevention system according to a first embodiment;
FIG. 4 is a diagram showing Configuration 3 of the endoscope fogging prevention system according to the first embodiment including Configurations 1 and 2 shown in FIG. 3;
FIG. 5A is a graph showing the relation between the measurement value of the temperature change of the temperature sensor and the estimated value of the temperature change caused by the heater during a normal time;
FIG. 5B is a graph showing the relation between the measurement value of the temperature change of the temperature sensor and the estimated value of the temperature change caused by the heater during a breakdown;
FIG. 6A is a flowchart illustrating an endoscope fogging prevention method according to the first embodiment;
FIG. 6B is one part of a flowchart illustrating a breakdown detection method according to the first embodiment including the fogging prevention operation method shown in FIG. 6A;
FIG. 6C is another part of the flowchart illustrating the breakdown detection method according to the first embodiment including the fogging prevention operation method shown in FIG. 6A;
FIG. 7A is a diagram showing the configuration of an endoscope fogging prevention system according to a second embodiment;
FIG. 7B is one part of a flowchart illustrating a breakdown detection method according to the second embodiment including the fogging prevention operation method according to the second embodiment;
FIG. 7C is another part of the flowchart illustrating the breakdown detection method according to the second embodiment including the fogging prevention operation method according to the second embodiment;
FIG. 8A is a diagram showing the configuration of an endoscope fogging prevention system according to a third embodiment;
FIG. 8B is a graph showing the relation between the measurement value of the temperature change of the temperature sensor and the estimated value of the temperature change caused by the heater according to the third embodiment;
FIG. 8C is one part of a flowchart illustrating a breakdown detection method according to the third embodiment including the fogging prevention operation method according to the third embodiment; and
FIG. 8D is another part of the flowchart illustrating the breakdown detection method according to the third embodiment including the fogging prevention operation method according to the second embodiment.

### Description of Embodiments

Hereinafter, embodiments of the present invention will be described in detail.

### [First Embodiment]

### [Configuration]

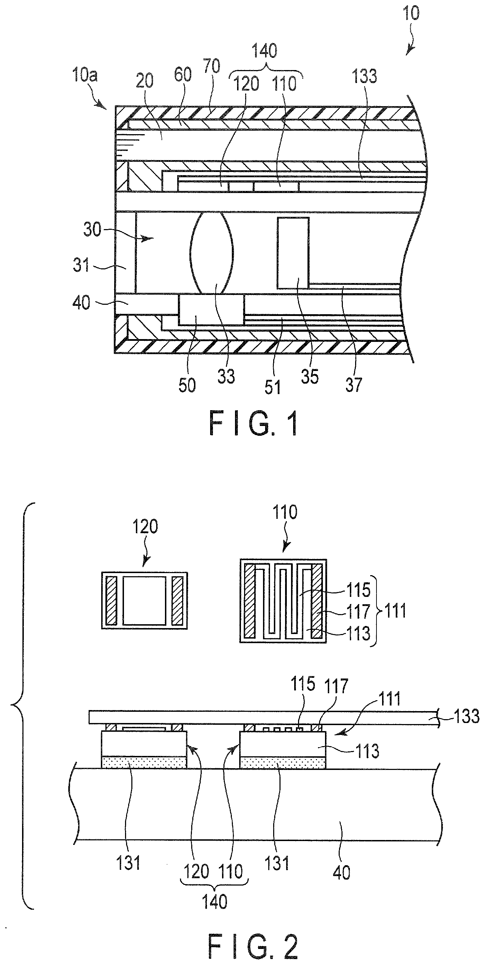

The first embodiment is described with reference to FIG. 1, FIG. 2, FIG. 3, FIG. 4, FIG. 5A, FIG. 5B, FIG. 6A, FIG. 6B, and FIG. 6C. In some of the drawings, components are not shown for clarity of diagrammatic representation.

### [Configuration of distal end portion 10a of endoscope]

As shown in FIG. 1, an unshown endoscope has an insertion portion 10. A distal end portion 10a of the insertion portion 10 has a light guide 20 which guides illumination light to irradiate the illumination light to an observation target, and an imaging unit 30 which images the observation target. The distal end portion 10a also has a lens frame 40 which holds the imaging unit 30, and a driver element 50 which is provided in the lens frame 40 and which drives a lens 33 of the imaging unit 30 to perform focusing or zooming.

The light guide 20 is inserted to an unshown connector of the endoscope via the insertion portion 10, an unshown operation portion of the endoscope, and an unshown universal cord of the endoscope. This connector is connected to an unshown light source apparatus so that the light guide 20 is connected to the light source apparatus. Consequently, the light is supplied to the light guide 20. The light guide 20 then emits the illumination light to the outside from a distal end portion of the light guide 20.

The imaging unit 30 has a lens cover 31 provided inside the distal end portion 10a to be exposed to the outside from a distal face of the distal end portion 10a, and the lens 33 provided behind the lens cover 31. The imaging unit 30 further has an image pickup element 35 provided behind the lens 33, and an imaging cable 37 which is connected to the image pickup element 35, which supplies electric power to the image pickup element 35 and which sends a control signal to control the image pickup element 35 to the image pickup element 35 and transmits a video signal obtained by the image pickup element 35.

The imaging cable 37 is inserted to the connector via the insertion portion 10, the operation portion, and the universal cord. This connector is connected to an unshown control apparatus which controls the endoscope, and the imaging cable 37 is thereby connected to the control apparatus. Thus, the electric power and the control signal to drive the image pickup element 35 are supplied to the imaging cable 37. The imaging cable 37 then supplies the electric power and the control signal to the image pickup element 35. This connector is connected to the control apparatus so that the video signal obtained by the image pickup element 35 is transmitted to the control apparatus.

No lens cover 31 may be provided, and the lens 33 may be directly exposed to the outside from the distal face of the distal end portion 10a. In the following explanation, at least one of the lens cover 31 and the lens 33 at the distal end portion 10a which is prevented from fogging when inserted into, for example, a body cavity is referred to as an optical member. The optical member has only to be provided, for example, inside the distal end portion 10a to be exposed to the outside from the distal face of the distal end portion 10a.

The driver element 50 has, for example, a motor. The driver element 50 is connected to a driver cable 51 which supplies the electric power to the driver element 50 and which sends a control signal to control the driver element 50 to the driver element 50.

The driver cable 51 is inserted to the connector via the insertion portion 10, the operation portion, and the universal cord. This connector is connected to the unshown control apparatus so that the driver cable 51 is connected to the control apparatus. Thus, the electric power and the control signal to drive the driver element 50 are supplied to the driver cable 51. The driver cable 51 then supplies the electric power and the control signal to the driver element 50.

The lens frame 40 is formed by, for example, a circular cylindrical member. The lens frame 40 houses the imaging unit 30 in the circular cylinder.

As shown in FIG. 1, the distal end portion 10a further has an inner frame 60 which holds the light guide 20 and the lens frame 40, and an outer frame 70 which covers the inner frame 60 and which functions as an outermost layer of the distal end portion 10a.

The inner frame 60 is made of, for example, a metal, and the outer frame 70 is made of, for example, a resin.

### [Fogging of optical member]

The above-mentioned endoscope having the distal end portion 10a is normally placed under an environment of managed temperature and humidity, for example, in a treatment room. Thus, the distal end portion 10a is exposed to such temperature and humidity before use. When the insertion portion 10 is inserted into the body cavity, the fogs occurs in the optical member such as the lens cover 31 due to, for example, the temperature difference between the room temperature and the body temperature or a high-humidity environment (a humidity of about 98% to about 100%) inside the body, thereby the imaging field of view significantly deteriorates.

### [Configuration 1 of endoscope fogging prevention system 100 (heater 110 and temperature sensor 120)]

Thus, as shown in FIG. 1, FIG. 2, FIG. 3, and FIG. 4, the endoscope and the unshown control apparatus which controls the endoscope are equipped with an endoscope fogging prevention system 100 to prevent the fogging of the endoscope. The endoscope fogging prevention system 100 has a heater 110 which is provided in, for example, the lens frame 40 and which is included in a heating section for heating the inside of the distal end portion 10a including the lens cover 31 via the lens frame 40 to prevent the fogging which occurs in the optical member such as the lens cover 31, and a temperature sensor 120 which is provided in, for example, the lens frame 40 and which is a temperature measurement section for measuring heat quantity information regarding the inside of the distal end portion 10a including the lens cover 31 via the lens frame 40. The heat quantity information will be described later.

As shown in FIG. 2, for example, a rear surface of the heater 110 and a rear surface of the temperature sensor 120 are joined to an outer circumferential surface of the lens frame 40 by an adhesive agent 131 having high heat conductivity. As shown in FIG. 1, the heater 110 and the temperature sensor 120 have only to be provided inside the distal end portion 10a. As shown in FIG. 1 and FIG. 2, the heater 110 and the temperature sensor 120 are mounted on a flexible substrate 133 by, for example, surface-mount technology. The heater 110 and the temperature sensor 120 are formed as a heater unit 140 mounted on the flexible substrate 133. The flexible substrate 133 is connected to an unshown cable which supplies the electric power and the control signal to the heater 110 and the temperature sensor 120 via the flexible substrate 133 and which transmits detection data detected by the temperature sensor 120. This cable is inserted to the connector via the insertion portion 10, the operation portion, and the universal cord. This connector is connected to the control apparatus so that the cable is connected to the control apparatus. Thus, the electric power and the control signal to drive the heater 110 and the temperature sensor 120 are supplied to the heater 110 and the temperature sensor 120. The cable then supplies the electric power and the control signal to the heater 110 and the temperature sensor 120. Moreover, this connector is connected to the control apparatus so that temperature data included in the detection data detected by the temperature sensor 120 is transmitted to the control apparatus.

As shown in FIG. 1 and FIG. 2, for example, the heater 110 is provided adjacent to the temperature sensor 120 in a longitudinal axis direction of the distal end portion 10a. For example, the heater 110 is provided a desired distance apart from the temperature sensor 120. For example, the heater 110 is provided farther from the lens cover 31 (the rear surface of the distal end portion 10a) than the temperature sensor 120. The positions where the heater 110 and the temperature sensor 120 are provided may be reversed. The positional relation between the heater 110 and the temperature sensor 120 is not particularly limited.

### [Heater 110]

The heater 110 heats the inside of the distal end portion 10a, for example, to such a temperature that the lens cover 31 is higher than the body temperature and that a living tissue is not burned. This temperature is, for example, about 38°C or more and 42°C or less.

As shown in FIG. 2, the heater 110 has, for example, a heating chip 111. This heating chip 111 has, for example, a ceramic substrate 113, a metallic resistance 115 provided on the substrate 113, and a pad 117 which is provided on the substrate 113 and which is electrically connected to the metallic resistance 115. The metallic resistance 115 is formed into a thin film shape or a paste state, and the pad 117 is formed as an electric current introduction terminal.

### [Temperature sensor 120]

The heat quantity information regarding the inside of the distal end portion 10a measured by the temperature sensor 120 comprises, for example, the temperature inside the distal end portion 10a formed based on a heat quantity of the heater 110. The temperature sensor 120 is formed by a ceramic substrate as a base in the same manner as the heating chip 111, or by a thermistor comprising a bulk.

### [Configuration 2 of endoscope fogging prevention system 100 • control unit 150]

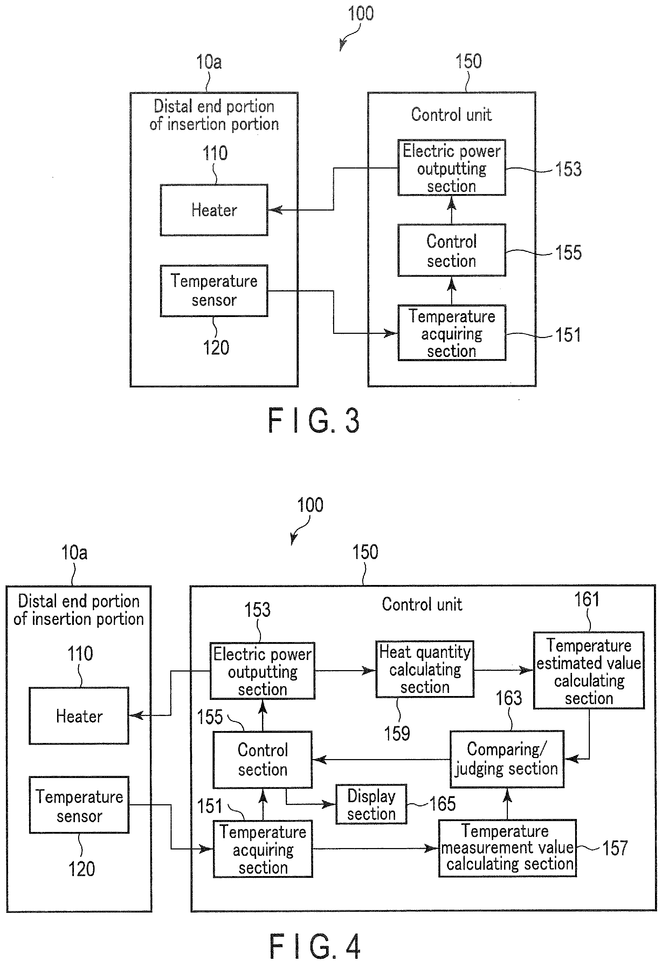

As shown in FIG. 3 and FIG. 4, the endoscope fogging prevention system 100 further has a control unit 150 which controls the driving of the heater 110 based on the temperature indicating the heat quantity information regarding the inside of the distal end portion 10a measured by the temperature sensor 120. The control unit 150 is, for example, separate from the endoscope. The control unit 150 is connected to, for example, the universal cord of the endoscope, and is provided in an unshown control apparatus which controls the endoscope.

As shown in FIG. 3 and FIG. 4, the control unit 150 has a temperature acquiring section 151 which acquires the actual temperature inside the distal end portion 10a measured by the temperature sensor 120, and an electric power outputting section 153 which outputs, to the heater 110, electric power (hereinafter referred to as heater driving electric power) necessary for the driving of the heater 110.

As shown in FIG. 3 and FIG. 4, the control unit 150 further has a control section 155 which calculates the difference between the temperature acquired by the temperature acquiring section 151 and a preset target temperature, calculates heater driving electric power that eliminates the difference based on the calculated difference, and controls the electric power outputting section 153 so that the electric power outputting section 153 outputs the calculated heater driving electric power to the heater 110. The target temperature is, for example, a temperature that prevents the fogging of an optical member such as the lens cover 31 by heating the optical member. The target temperature is a temperature less than or equal to the temperature at which the outer frame 70 that is an outermost part of the distal end portion 10a does not burn the living tissue. The target temperature can be, for example, suitably adjusted in a desired manner by, for example, the control unit 150. The target temperature is previously recorded in, for example, an unshown recording section provided in the control unit 150.

The temperature, which is an acquisition result acquired by the temperature acquiring section 151, is recorded in the unshown recording section. The temperature acquiring section 151 acquires the temperature, for example, at a desired timing or for a desired period.

The temperature measured by the temperature sensor 120 is fed back to the control unit 150. The temperature inside the distal end portion 10a is high accurately controlled by repeated feedback so that the heating temperature of the heater 110 will be set to the desired temperature. The control method of the heater 110 is, for example, on-off control, PWM control, or PID control.

### [Example of breakdown of endoscope fogging prevention system 100]

An example of the breakdown of the endoscope fogging prevention system 100 is the breakdown of the temperature sensor 120. This breakdown shows a characteristic change of the temperature sensor 120 including, for example, the deterioration of the temperature sensor 120 with time or the breakage of the temperature sensor 120 caused by external factors such as static electricity. This leads to a difference between the actual temperature inside the distal end portion 10a and the temperature measured by the temperature sensor 120 (the temperature acquired by the temperature acquiring section 151).

As described above, the control section 155 calculates the heater driving electric power based on the temperature acquired by the temperature acquiring section 151. Thus, if the temperature measured by the temperature sensor 120 is different from the actual temperature inside the distal end portion 10a, there is fear that the heater 110 may heat the inside of the distal end portion 10a to the target temperature or more or to the target temperature or less.

Another example of the breakdown is a void generated in the adhesive agent 131 or partial detachment of the heater 110 or the temperature sensor 120 from the lens frame 40. As a result, heat conducting properties change between the lens frame 40 and the heater 110 and between the lens frame 40 and the temperature sensor 120. Thus, there is fear that the heat generated from the heater 110 may not be transmitted to the lens frame 40 and that the temperature sensor 120 may not measure the actual temperature inside the distal end portion 10a. As described above, there is fear that the heater 110 may heat the inside of the distal end portion 10a to the target temperature or more or to the target temperature or less.

Yet another example of the breakdown is the breakdown of the heater 110. This breakdown shows a characteristic change of the heater 110 including, for example, the deterioration of the heater 110 with time or the breakage of the heater 110 caused by external factors such as static electricity.

### [Configuration 3 of endoscope fogging prevention system 100]

Thus, the endoscope fogging prevention system 100 judges the operating characteristics of the heater 110 and the operating characteristics of the temperature sensor 120 based on the heat quantity information regarding the inside of the distal end portion 10a, and, for example, deactivates the heater 110 when the judgment result is out of a desired range. As described above, the heat quantity information is formed by, for example, the heat quantity of the heater 110. The heat quantity affects, for example, the internal temperature of the distal end portion 10a. The heat quantity of the heater 110 is formed based on an integration value in which a control time and control electric power for the heater 110 are integrated, that is, based on time integration of the control electric power. The heat quantity can also be found by converting a controlled variable for the heater 110 of, for example, a voltage or an electric current to the control electric power using other parameters. That is, the heat quantity of the heater 110 is formed based on the integration value in which the control time and the controlled variable for the heater 110 are integrated. The control electric power for the heater 110 is, for example, the heater driving electric power necessary for the driving of the heater 110.

Specifically, as shown in FIG. 4, the control unit 150 of the endoscope fogging prevention system 100 has a temperature measurement value calculating section 157 which calculates a measurement value of a temperature change at a desired time based on the measured temperature acquired by the temperature acquiring section 151. This measured temperature is the temperature actually measured by the temperature sensor 120.

The control unit 150 further has a heat quantity calculating section 159 which integrates the control time and the heater driving electric power for a desired time to calculate a heat quantity (integration value) of the heater 110, and a temperature estimated value calculating section 161 which calculates an estimated value of the temperature change of the heater 110 at a desired time based on the heat quantity (integration value) of the heater 110 calculated by the heat quantity calculating section 159.

The control unit 150 further has a comparing/judging section 163 which compares the measurement value with the estimated value and judges whether the difference between the measurement value and the estimated value is within a desired range. The control unit 150 further has a display section 165 which displays a warning of deactivation when the comparing/judging section 163 judges that the difference between the measurement value and the estimated value is not within the desired range and thereby the control section 155 deactivates the heater 110 and the temperature sensor 120.

### [Temperature measurement value calculating section 157]

In the temperature measurement value calculating section 157, the measurement value of the temperature change shows, for example, the difference between a temperature A1 and a temperature A2. As described above, the temperature acquiring section 151 acquires a temperature at a desired timing. t=0 represents the timing for the temperature acquiring section 151 to acquire a temperature for the first time, wherein t is the time at which the temperature acquiring section 151 acquires the temperature. The temperature A1 represents the temperature at this moment, and is an actually measured value. t=T represents the timing for the temperature acquiring section 151 to acquire a temperature after a desired time has elapsed since the first acquisition of the temperature, wherein T is a desired time elapsed since the first time. The temperature A2 represents the temperature at this moment, and is an actually measured value.

Thus, the measurement value is the actually measured value which is the difference between the measured temperature A1 and the measured temperature A2. In other words, the measurement value is the actually measured value of the temperature change at a desired time calculated based on the temperatures A1 and A2 inside the distal end portion 10a that have been acquired by the temperature acquiring section 151 from the temperature measurement section.

The timing for the temperature acquiring section 151 to acquire a temperature for the first time may be, for example, a immediately after the start of heating or a desired timing after the elapse of a certain period of time since the start of heating, and can be adjusted to a desired timing.

### [Heat quantity calculating section 159]

The heat quantity calculating section 159 calculates a heat quantity in response to, for example, the heater driving electric power from the electric power outputting section 153 or the control section 155. The heat quantity calculating section 159 also calculates the heat quantity of the heater 110 synchronously with the above-mentioned t. That is, the heat quantity calculating section 159 calculates a heat quantity (integration value C) generated in the heater 110 between t=0 and t=T. The heat quantity of the heater 110 calculated by the heat quantity calculating section 159 is recorded in the unshown recording section.

If the internal structure of the distal end portion 10a is determined in advance, the temperature change of the distal end portion 10a at a desired time is estimated based on the heat quantity of the heater 110.

### [Temperature estimated value calculating section 161]

In the temperature estimated value calculating section 161, the estimated value shows an estimated value of the temperature change at a desired time calculated based on the heater driving electric power. In the present embodiment, the estimated value shows an estimated value of the temperature change at a desired time calculated based on the integration value C.

In the temperature estimated value calculating section 161, a conversion coefficient for converting the heat quantity of the heater 110 to the temperature change can be theoretically found, for example, from a physical property value of the structure of the distal end portion 10a, or can be experimentally found. A general method such as an approximate expression or a conversion table can be used as the conversion method.

### [Comparing/judging section 163]

In the comparing/judging section 163, the desired range can be adjusted in a desired manner in consideration of a disturbance, for example, a measurement error or a variation caused.

When the comparing/judging section 163 judges that the difference between the measurement value and the estimated value is within the desired range, the comparing/judging section 163 judges that the heater 110 and the temperature sensor 120 are normal, and outputs the judgment result to the control section 155. The control section 155 calculates the difference between the temperature (measurement value) acquired by the temperature acquiring section 151 and a preset desired temperature in response to the judgment result, calculates the heater driving electric power, controls the electric power outputting section 153, and then controls the heater 110 and the temperature sensor 120.

When the comparing/judging section 163 judges that the difference between the measurement value and the estimated value is not within the desired range, the comparing/judging section 163 judges that the measurement value has diverged from the actual temperature inside the distal end portion 10a and that at least one of the heater 110 and the temperature sensor 120 has broken down, and outputs the judgment result to the control section 155. In response to this judgment result, the control section 155 deactivates the heater 110 and the temperature sensor 120.

Thus, the endoscope fogging prevention system 100 compares the measurement value with the estimated value, and judges whether the difference between the measurement value and the estimated value is within the desired range, and thereby judges the operating characteristics of the heater 110 and the operating characteristics of the temperature sensor 120. The operating characteristics are, for example, normal or a breakdown. The endoscope fogging prevention system 100 always conducts this judgment before the use of the endoscope or during the use of the endoscope.

### [Relation between time t and heater driving electric power]

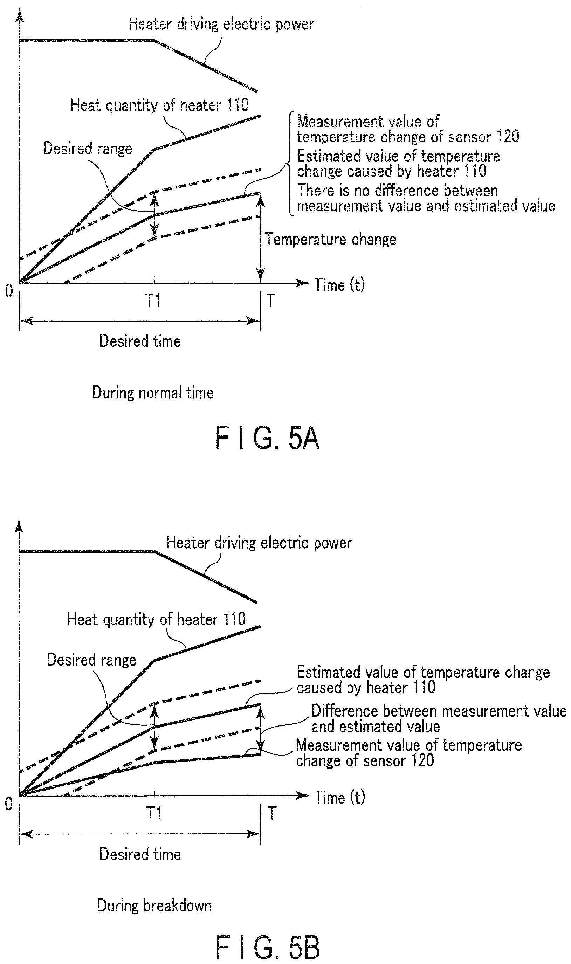

Next, the relation between the time t and the heater driving electric power is described with reference to FIG. 5A and FIG. 5B.

At t=0, the temperature sensor 120 starts the measurement of the temperature inside the distal end portion 10a, and the temperature acquiring section 151 starts the acquisition of the temperature. Further, the control section 155 calculates the difference between the temperature acquired by the temperature acquiring section 151 and the preset desired temperature. The control section 155 then calculates the heater driving electric power that eliminates the difference based on the calculated difference.

For example, when the temperature of the distal end portion 10a is low enough, the set maximum (constant) heater driving electric power is supplied to the heater 110 from the electric power outputting section 153 so that the heater 110 will output the set maximum heat quantity. The heater 110 then heats the inside of the distal end portion 10a.

After the elapse of a desired time (t=T1), the inside is sufficiently warmed, and the difference becomes smaller. The control section 155 then calculates that the difference becomes smaller. Accordingly, the heat quantity of the heater 110 needs to be suppressed. Thus, the control section 155 recalculates the heater driving electric power which is less than the above-mentioned constant heater driving electric power. This heater driving electric power is then supplied to the heater 110 from the electric power outputting section 153. In this case, for example, the supply amount of the heater driving electric power to the heater 110 from the electric power outputting section 153 gradually decreases.

### [Relation between heater driving electric power, heat quantity of heater 110, measurement value, and estimated value]

Next, the relation between the heater driving electric power, the heat quantity of heater 110, the measurement value, and the estimated value is described with reference to FIG. 5A and FIG. 5B.

As shown in FIG. 5A and FIG. 5B, the heater driving electric power is constant until 0<t<T1 in the above-mentioned situation, so that the integration value which is the heat quantity of heater 110 increases with a constant inclination along with the elapse of time.

As shown in FIG. 5A and FIG. 5B, when T1<t, the heater driving electric power gradually decreases, so that the integration value which is the heat quantity of the heater 110 increases with an inclination smaller than that when 0<t<T1 every time a desired time elapses.

As shown in FIG. 5A and FIG. 5B, in the above-mentioned situation, the estimated value of the temperature change of the heater 110 also increases with a substantially similar inclination as that of the heat quantity of the heater 110.

As shown in FIG. 5A, in the above-mentioned situation, the measurement value of the temperature change of the heater 110 increases with a substantially similar inclination as that of the heat quantity of the heater 110.

When there is no difference between the measurement value and the estimated value as shown in FIG. 5A, i.e., when the measurement value corresponds to the estimated value, or when there is a slight difference between the measurement value and the estimated value, it is judged that the difference is within the desired range and that the measurement value is equal to the actual temperature inside the distal end portion 10a. Thus, the comparing/judging section 163 judges that the heater 110 and the temperature sensor 120 are normal.

As shown in FIG. 5B, when there is a great difference between the measurement value and the estimated value, it is judged that the difference is not within the desired range and that the divergent occurs in the actual temperature inside the distal end portion 10a. Thus, the comparing/judging section 163 judges that at least one of the heater 110 and the temperature sensor 120 has broken down.

### [Function 1]

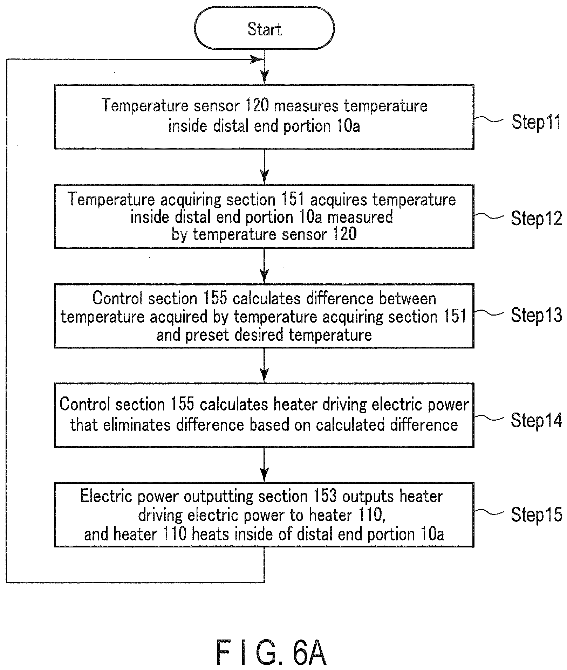

Next, the fogging prevention operation method according to the present embodiment is described with reference to a flowchart shown in FIG. 6A.

The temperature sensor 120 measures the temperature inside the distal end portion 10a (Step 11).

The temperature acquiring section 151 then acquires the temperature inside the distal end portion 10a measured by the temperature sensor 120 (Step 12).

The control section 155 then calculates the difference between the temperature acquired by the temperature acquiring section 151 and the preset desired temperature (Step 13).

The control section 155 then calculates the heater driving electric power that eliminates the difference based on the calculated difference (Step 14).

The control section 155 then controls the electric power outputting section 153 so that the electric power outputting section 153 will output the heater driving electric power calculated by the control section 155 to the heater 110. The electric power outputting section 153 outputs the heater driving electric power to the heater 110, and the heater 110 heats the inside of the distal end portion 10a (Step 15). The operation then returns to Step 11.

The operations in Step 11 to Step 15 are repeated, and the fogging of the optical member such as the lens cover 31 is prevented.

### [Function 2]

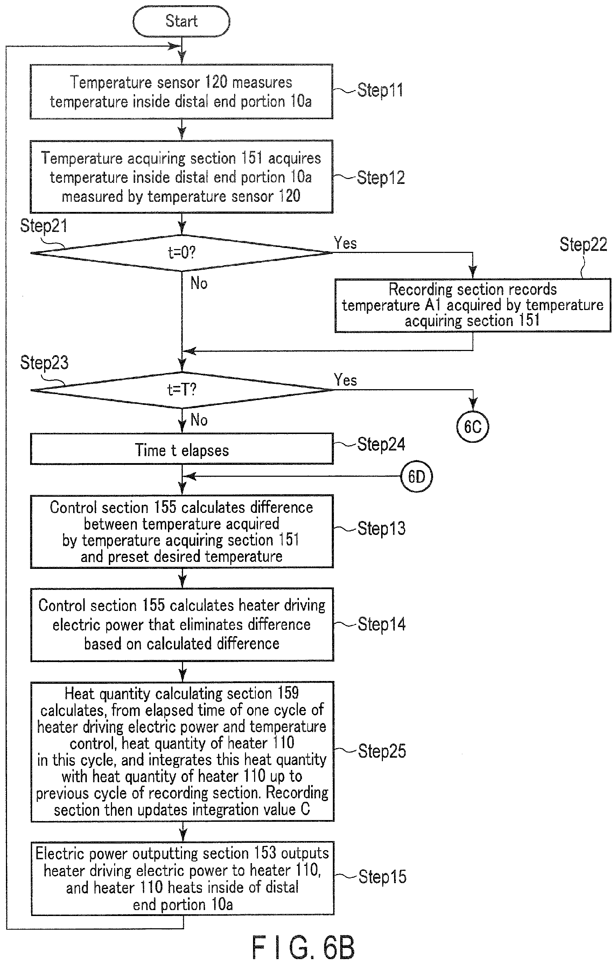

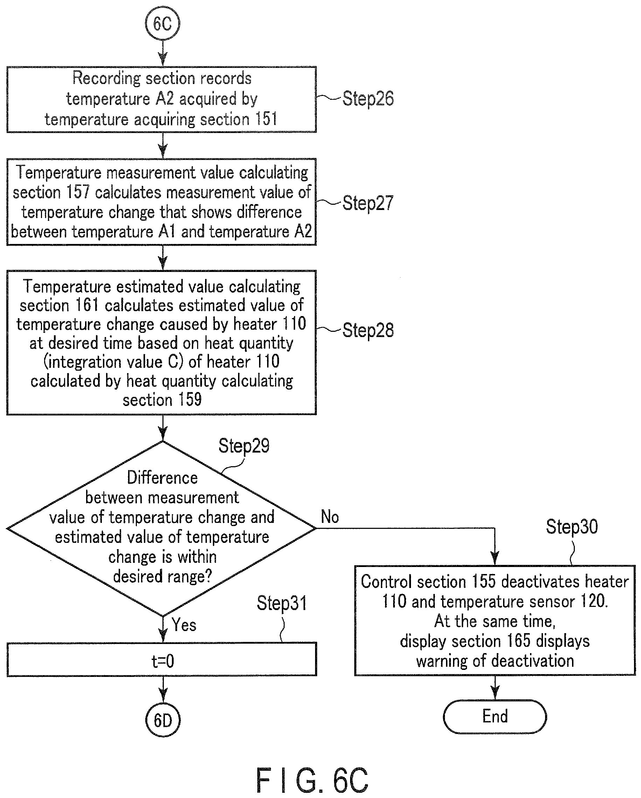

Next, a method of detecting the breakdown of at least one of the heater 110 and the temperature sensor 120 including the fogging prevention operation method according to the present embodiment is described with reference to a flowchart shown in FIG. 6B and FIG. 6C. The same Steps as those shown in FIG. 6A are provided with the same numbers.

As shown in FIG. 6B, the temperature sensor 120 measures the temperature A1 inside the distal end portion 10a (Step 11).

The temperature acquiring section 151 then acquires the temperature A1 inside the distal end portion 10a measured by the temperature sensor 120 (Step 12).

The control section 155 then judges whether t=0 (Step 21) .

When t=0 (Step 21: Yes), the recording section records the temperature A1 acquired by the temperature acquiring section 151 (Step 22). When t=0 (Step 21: No), the operation then returns to Step 23.

The control section 155 then judges whether t=T (Step 23) .

When t is not equal to T (Step 23: No), the time t elapses (Step 24).

The control section 155 then calculates the difference between the temperature acquired by the temperature acquiring section 151 and the preset desired temperature (Step 13).

The control section 155 then calculates the heater driving electric power that eliminates the difference based on the calculated difference (Step 14).

The heat quantity calculating section 159 then calculates, from an elapsed time of one cycle of the heater driving electric power and the temperature control, a heat quantity of the heater 110 in this cycle, and integrates this heat quantity with the heat quantity of the heater 110 up to the previous cycle of the recording section. The recording section then updates the integration value C (Step 25).

The control section 155 then controls the electric power outputting section 153 so that the electric power outputting section 153 will output the heater driving electric power calculated by the control section 155 to the heater 110. The electric power outputting section 153 outputs the heater driving electric power to the heater 110, and the heater 110 heats the inside of the distal end portion 10a (Step 15).

As a result, the fogging of the optical member such as the lens cover 31 is prevented.

When the desired time T has elapsed, the operation then returns to Step 11, and the temperature sensor 120 measures the temperature inside the distal end portion 10a (Step 11).

The temperature acquiring section 151 then acquires the temperature inside the distal end portion 10a measured by the temperature sensor 120 (Step 12).

As described above, the desired time T has elapsed, so that t=T. Thus, in Steps 11 and 12, the temperature sensor 120 measures the temperature A2 at this time T, and the temperature acquiring section 151 acquires the temperature A2 at this time T.

Since t=T (Step 21: No, Step 23: Yes), the recording section then records the temperature A2 acquired by the temperature acquiring section 151 as shown in FIG. 6C (Step 26) .

As shown in FIG. 6C, the temperature measurement value calculating section 157 then calculates a measurement value of a temperature change that shows the difference between the temperature A1 and the temperature A2 (Step 27).

The temperature estimated value calculating section 161 then calculates an estimated value of the temperature change caused by the heater 110 at a desired time based on the heat quantity (integration value C) of the heater 110 calculated by the heat quantity calculating section 159 (Step 28).

The comparing/judging section 163 then compares the measurement value of the temperature change with the estimated value of the temperature change, and judges whether the difference between the measurement value of the temperature change and the estimated value of the temperature change is within the desired range (Step 29).

When the comparing/judging section 163 judges that the difference between the measurement value and the estimated value is not within the desired range (Step 29: No), the comparing/judging section 163 judges that at least one of the heater 110 and the temperature sensor 120 has broken down, and outputs the judgment result to the control section 155. In response to this judgment result, the control section 155 deactivates, for example, the heater 110 and the temperature sensor 120. At the same time, the display section 165 displays a warning of deactivation (Step 30).

When the comparing/judging section 163 judges that the difference between the measurement value and the estimated value is within the desired range (Step 29: Yes), the comparing/judging section 163 judges that the heater 110 and the temperature sensor 120 are normally operating. The control section 155 then controls so that t=0 to set off one cycle of the breakdown judgment (Step 31).

The operation then proceeds to Step 13 shown in FIG. 6B.

After Step 13, the operation sequentially proceeds to Step 14, Step 25, and Step 15, and returns to Step 11. Since t=0 in Step 31 as described above, the temperature sensor 120 measures the temperature A1 in Step 11, the temperature acquiring section 151 acquires the temperature A1 in Step 12, the operation sequentially proceeds to Step 21, Step 22, and Step 23, and the operations described above are repeated.

The operations described above are always repeated before the use of the endoscope or during the use of the endoscope. Thus, before the use of the endoscope or during the use of the endoscope, the fogging of the optical member such as the lens cover 31 is always prevented, and the breakdown of at least one of the heater 110 and the temperature sensor 120 is detected at the same time.

### [Advantageous effects]

Thus, according to the present embodiment, in Step 29, the comparing/judging section 163 compares the measurement value of the temperature change with the estimated value of the temperature change, and judges whether the difference between the measurement value and the estimated value is within the desired range. Thus, according to the present embodiment, it is possible to high accurately detect whether at least one of the heater 110 and the temperature sensor 120 has broken down or is normally operating.

According to the present embodiment, in Step 13, the control section 155 calculates the difference between the temperature acquired by the temperature acquiring section 151 and the preset desired temperature. According to the present embodiment, in Step 14, the control section 155 calculates the heater driving electric power that eliminates the difference based on the calculated difference. Thus, according to the present embodiment, it is possible to detect the above-mentioned breakdown, and the fogging of the optical member such as the lens cover 31 at the same time.

According to the present embodiment, the heat quantity information is formed based on the heat quantity of the heater 110, and this heat quantity is formed based on the integration value in which the time and the heater driving electric power are integrated. Thus, according to the present embodiment, it is possible to high accurately calculate the estimated value, high accurately calculate the difference between the measurement value and the estimated value, and certainly and high accurately detect whether at least one of the heater 110 and the temperature sensor 120 has broken down or is normally operating.

According to the present embodiment, it is possible to detect before the use of the endoscope whether at least one of the heater 110 and the temperature sensor 120 has broken down or is normally operating by conducting a judgment before the use of the endoscope. According to the present embodiment, it is also possible to always judge during the use of the endoscope whether at least one of the heater 110 and the temperature sensor 120 has broken down or is normally operating by always conducting a judgment during the use of the endoscope.

Although the heat quantity calculating section 159 integrates the heater driving electric power with a desired time included in the controlled variable to calculate a heat quantity (integration value) of the heater 110 according to the present embodiment, the present invention does not need to be limited to this. When the resistance temperature coefficient of the heater 110 is low enough, the controlled variable may include the voltage to be applied to the heater 110, the electric current to be applied to the heater 110, or the resistance value of the heater 110. Thus, the controlled variable is at least one of the electric power, the voltage, and the electric current. The integration section may integrate one of the electric power, the voltage, and the electric current with the desired time to calculate an integration value corresponding to a heat quantity of the heater 110.

In the desired time, for example, when the heater driving electric power is high, i.e., when the heat quantity (integration value) of the heater 110 is higher, the desired time may be shorter. For example, when the heater driving electric power is low, i.e., when the heat quantity (integration value) of the heater 110 is lower, the desired time may be longer. Thus, according to the present embodiment, it is possible to efficiently reduce judgment errors.

### [Second Embodiment]

### [Configuration]

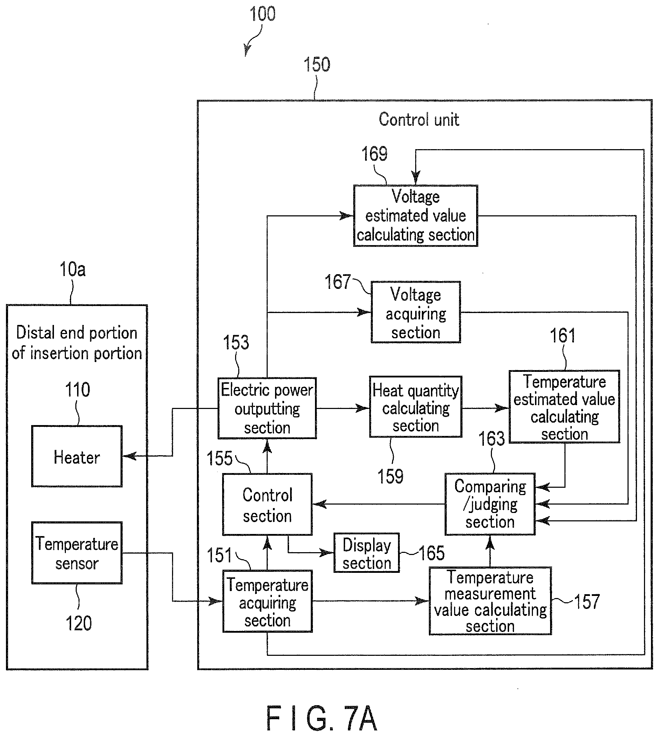

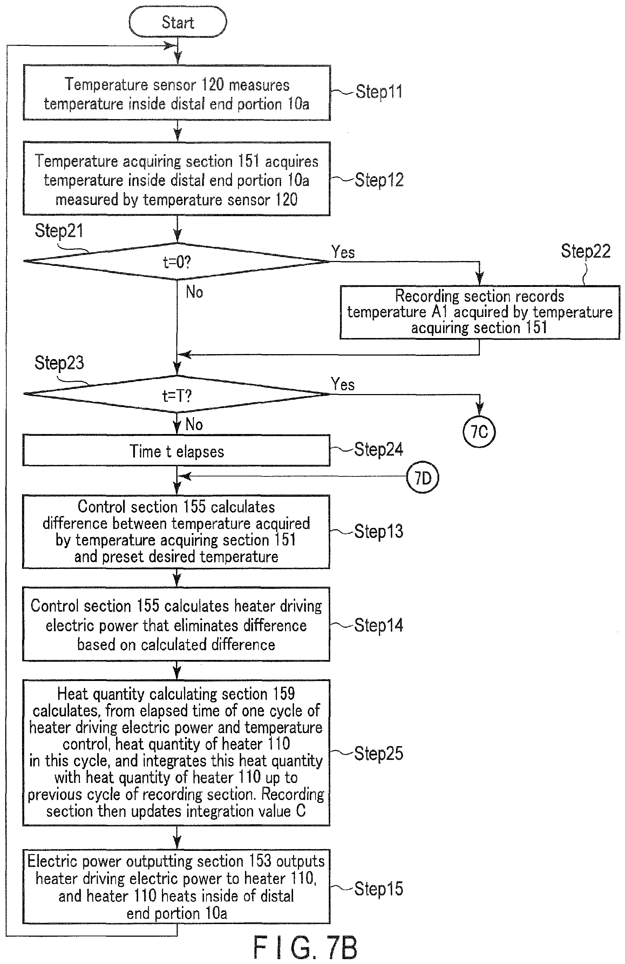

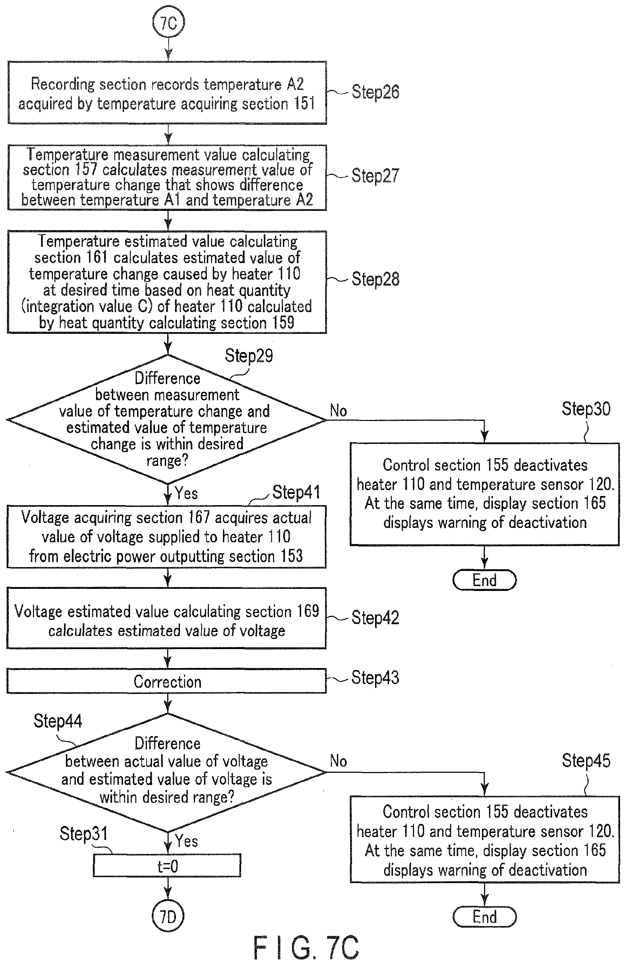

In the present embodiment, configurations different from the configurations according to the first embodiment are only described below with reference to FIG. 7A, FIG. 7B, and FIG. 7C.

### [Example of breakdown of fogging prevention system 100 in the present embodiment]

One example of a breakdown is the breakdown of the heater 110. This breakdown shows a change of the characteristics, for example, the resistance value of the heater 110 including, for example, the deterioration of the heater 110 with time or the breakage of the heater 110 caused by external factors such as static electricity. In this instance, the heating state of the distal end portion 10a heated by the heater 110 and the temperature measured by the temperature sensor 120 are substantially the same as those in the normal state. Thus, when the heater 110 is controlled by the heater driving electric power, the heater 110 does not break down, and behaves as if normally operating. However, since the voltage value and the electric current value to be applied to the heater 110 change from those in the normal state, there is a fear that the heater 110 may not show desired performance when the heater 110 is controlled by the electric current and the voltage. Such a breakdown of the heater 110 deteriorates the state with accelerating speed, thus detecting the breakdown in an initial condition is desired.

Therefore, when the heater 110 is of a resistance heating type, the resistance characteristics of the heater 110 change if the heater 110 breaks down. For example, even if the resistance value has increased as a result of a breakdown, the heat quantity of the heater 110 that has broken down is the same as that during a normal time regardless of the resistance value of the heater 110 when the heater 110 is controlled by the heater driving electric power supplied thereto.

Thus, in the first embodiment, there is a fear that the estimated value of the temperature change may remain the same during the normal time and during a breakdown even if the estimated value of the temperature change is calculated based on the heat quantity (integration value) of the heater 110. Therefore, there is a fear that the comparing/judging section 163 may judge even the breakdown to be normal even if the comparing/judging section 163 compares the measurement value of the temperature change with the estimated value of the temperature change.

However, even if the heater driving electric power supplied to the heater 110 does not change, the voltage value and the electric current value to be applied to the heater 110 change in response to the change of the resistance value of the heater 110. The present embodiment focuses on this point, for example.

### [Configuration of endoscope fogging prevention system 100]

Thus, as shown in FIG. 7A, the endoscope fogging prevention system 100 further has a voltage acquiring section 167 which acquires an actual value of the voltage supplied to the heater 110 from the electric power outputting section 153, and a voltage estimated value calculating section 169 which calculates an estimated value of a voltage that is supplied to the heater 110 from the electric power outputting section 153 and that is proper for the current heater driving electric power and temperature based on, for example, the heater driving electric power included in the controlled variable and the temperature acquired by the temperature acquiring section 151 included in the heat quantity information. The actual value is the actually measured value of the voltage.

The comparing/judging section 163 according to the present embodiment compares the difference between the actual value of the voltage and the estimated value of the voltage, and further judges whether the difference between the actual value of the voltage and the estimated value of the voltage is within the desired range. In the comparing/judging section 163, the desired range can be adjusted in a desired manner in consideration of a measurement error or a variation caused by, for example, disturbance.

When the comparing/judging section 163 judges that the difference between the measurement value of the temperature change and the estimated value of the temperature change is within the desired range and that the difference between the actual value of the voltage and the estimated value of the voltage is within the desired range, the comparing/judging section 163 judges that the heater 110 and the temperature sensor 120 are normal, and outputs the judgment result to the control section 155. The control section 155 calculates the difference between the temperature acquired by the temperature acquiring section 151 and the preset desired temperature in response to the judgment result as described above, calculates the heater driving electric power, controls the electric power outputting section 153, and then controls the heater 110 and the temperature sensor 120.

When the comparing/judging section 163 judges that the difference between the measurement value of the temperature change and the estimated value of the temperature change is within the desired range and that the difference between the actual value of the voltage and the estimated value of the voltage is not within the desired range, the comparing/judging section 163 judges that the heater 110 has broken down, and outputs the judgment result to the control section 155. In response to this judgment result, the control section 155 deactivates the heater 110 and the temperature sensor 120.

When the comparing/judging section 163 judges that the difference between the measurement value of the temperature change and the estimated value of the temperature change is not within the desired range, the comparing/judging section 163 judges that the heater 110 or the temperature sensor 120 has broken down, and outputs the judgment result to the control section 155. In response to this judgment result, the control section 155 deactivates the heater 110 and the temperature sensor 120.

That is, the fogging prevention system 100 compares the actual value of the voltage to the heater 110 with the estimated value of the voltage calculated based on the heat quantity information, and judges whether the difference between the actual value of the voltage and the estimated value of the voltage is within the desired range, and thereby judges the operating characteristics of the heater 110 and the operating characteristics of the temperature sensor 120. The operating characteristics indicate, for example, normal or breakdown. The fogging prevention system 100 always conducts this judgment before the use of the endoscope or during the use of the endoscope.

### [Function]

Next, a method of accurately detecting the breakdown of the heater 110 including the fogging prevention operation method according to the present embodiment is described with reference to a flowchart shown in FIG. 7B and FIG. 7C.

The operations in Step 11 to Step 15 and in Step 21 to Step 31 are similar to those in the first embodiment and are therefore not described.

As shown in FIG. 7B, the operation in Step 11, the operation in Step 12, and the operation in Step 21 are sequentially performed.

If yes in Step 21, the operation in Step 22 and the operation in Step 23 are sequentially performed. If no in Step 21, the operation in Step 23 is performed.

If no in Step 23, the operation in Step 24, the operation in Step 13, the operation in Step 14, the operation in Step 25, and the operation in Step 15 are sequentially performed, and the operation returns to Step 11.

If yes in Step 23, the operation in Step 26, the operation in Step 27, the operation in Step 28, and the operation in Step 29 are sequentially performed as shown in FIG. 7C. If no in Step 29, the operation in Step 30 is performed.

As shown in FIG. 7C, if yes in Step 29, the voltage acquiring section 167 acquires an actual value of the voltage supplied to the heater 110 from the electric power outputting section 153 (Step 41).

The voltage estimated value calculating section 169 then calculates an estimated value of the voltage based on the heater driving electric power (Step 42).

When the resistance temperature coefficient of the heater 110 is relatively high, the resistance value then changes with the temperature. Thus, the voltage value in the normal state is corrected based on the temperature acquired by the temperature acquiring section 151 to separate the resistance value change caused by the temperature change and the resistance value change caused by the characteristic change.

When the resistance value change caused by the resistance temperature coefficient of the heater 110 is negligibly small in a temperature zone in which the endoscope is used, it is not necessary to make correction using temperature information. The correction is made by, for example, the voltage estimated value calculating section 169 (Step 43).

The comparing/judging section 163 then compares the actual value of the voltage with the estimated value of the voltage, and judges whether the difference between the actual value and the estimated value is within the desired range (Step 44).

When the comparing/judging section 163 judges that the difference between the actual value and the estimated value is not within the desired range (Step 44: No), the comparing/judging section 163 judges that the heater 110 has broken down, and outputs the judgment result to the control section 155. In response to this judgment result, the control section 155 deactivates the heater 110 and the temperature sensor 120. At the same time, the display section 165 displays a warning of deactivation (Step 45).

When the comparing/judging section 163 judges that the difference between the actual value and the estimated value is within the desired range (Step 44: Yes), the comparing/judging section 163 judges that the heater 110 and the temperature sensor 120 are normally operating. The operation then proceeds to Step 31.

### [Advantageous effects]

Thus, according to the present embodiment, in Step 44, the comparing/judging section 163 compares the actual value of the voltage with the estimated value of the voltage, and judges whether the difference between the actual value and the estimated value is within the desired range. Thus, according to the present embodiment, it is possible to more accurately detect whether the heater 110 has broken down or is normally operating.

Although the voltage value has been described by way of example in the present embodiment, the present invention does not need to be limited to this, and the electric current value may be used. That is, the fogging prevention system 100 compares the actual value of the electric current to the heater 110 with the estimated value of the electric current calculated based on the heat quantity information, and judges whether the difference between the actual value of the electric current and the estimated value of the electric current is within the desired range, and thereby judges the operating characteristics of the heater 110 and the operating characteristics of the temperature sensor 120. The operating characteristics indicate, for example, normal or breakdown. The fogging prevention system 100 always conducts this judgment before the use of the endoscope or during the use of the endoscope. The actual value may be a controlled variable, that is, the actually measured value of the electric power, the voltage, or the electric current.

Thus, the fogging prevention system 100 compares the actual value of the voltage or electric current included in the controlled variable to the heater 110 with the estimated value of the voltage or electric current included in the controlled variable calculated based on the heater driving electric power included in the controlled variable and the temperature acquired by the temperature acquiring section 151 included in the heat quantity information, and judges whether the difference between the actual value and the estimated value is within the desired range, and thereby judges the operating characteristics of the heater 110 and the operating characteristics of the temperature sensor 120.

### [Third Embodiment]

### [Configuration]

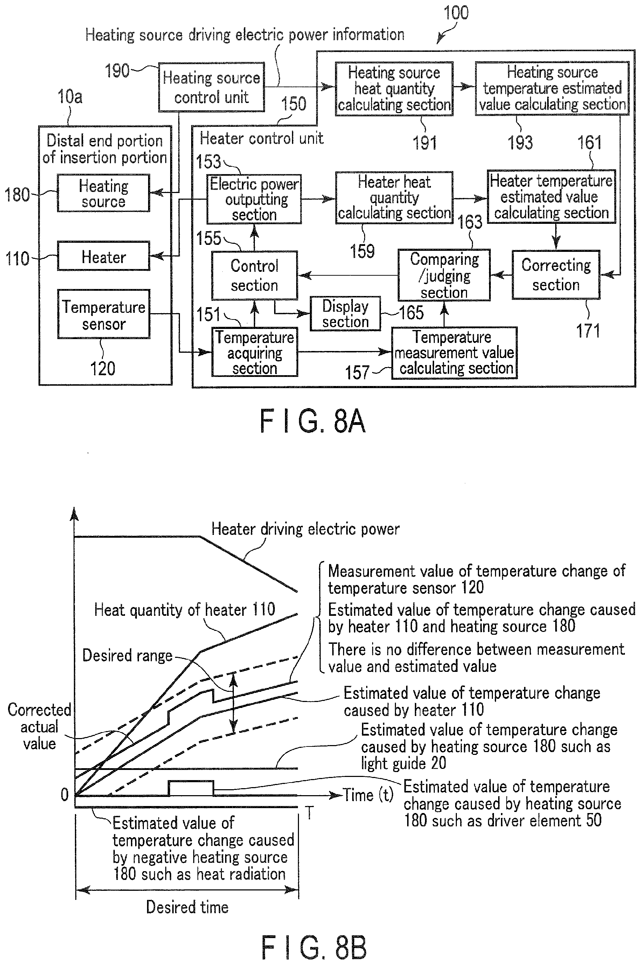

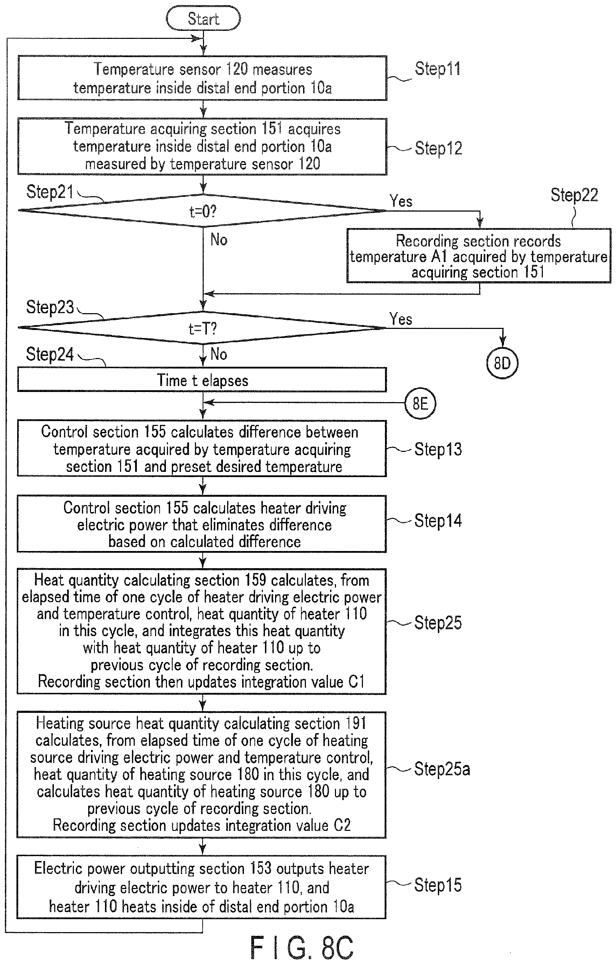

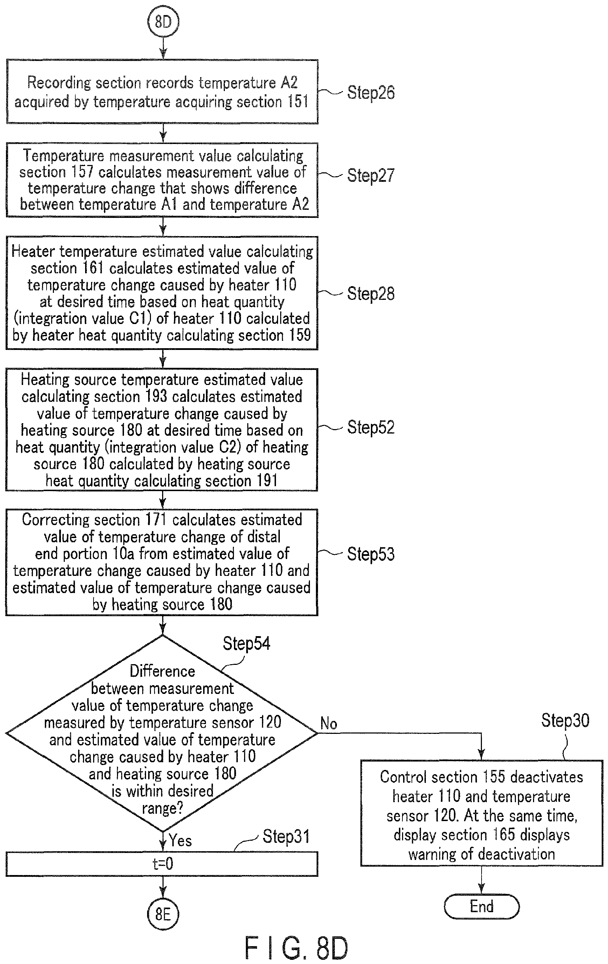

In the present embodiment, configurations different from the configurations according to the first embodiment are only described below with reference to FIG. 8A, FIG. 8B, FIG. 8C, and FIG. 8D.

In the present embodiment, the heating section has the heater 110, and a heating source 180 provided inside the distal end portion 10a. The heating source 180 has at least one of the light guide 20 which is a light source member, the imaging unit 30, and the driver element 50 which is a driving member to drive the imaging unit 30. The heating source 180 is controlled by a heating source control unit 190 such as the light source apparatus or the control apparatus. Heat radiation from the distal end portion 10a can also be considered as a negative heating source. In this case, the heat quantity information regarding the inside of the distal end portion 10a measured by the temperature sensor 120 according to the present embodiment shows, for example, the sum of temperature changes inside the distal end portion 10a formed based on the heat quantity of the heater 110 and the heat quantity of the heating source 180. The heat quantity of the heating source 180 is formed based on, for example, an integration value in which the control time and the controlled variable for the heating source 180 are integrated. This controlled variable is, for example, electric power (hereinafter referred to as heating source driving electric power) necessary for the driving of the heating source 180.

In the present embodiment, the fogging prevention system 100 calculates a heat quantity in which the heater driving electric power is integrated and a heat quantity in which the heating source driving electric power is integrated by a heater heat quantity calculating section 159 which calculates a heat quantity of the heater 110 and by a heating source heat quantity calculating section 191 which calculates a heat quantity of the heating source 180 based on the control time of the heating source 180 and driving electric power information for the heating source 180 output from the heating source control unit 190. A heater temperature estimated value calculating section 161 calculates an estimated value of the temperature change of the distal end portion 10a caused by the heater 110 calculated by the heat quantity calculating section 159, and a heating source temperature estimated value calculating section 193 calculates an estimated value of the temperature change of the distal end portion 10a caused by the heating source calculated by the heating source heat quantity calculating section 191.

From these estimated values, a correcting section 171 calculates an estimated value of the temperature change of the distal end portion 10a at a desired time.

As shown in FIG. 8B, when the heating source 180 is always driven as the light source member, the estimated value can be considered to be constant. When the heating source 180 is only driven by desired timing as the driver element 50 as shown in FIG. 8B, the estimated value is only calculated by driving timing.

The comparing/judging section 163 compares the measurement value of the temperature change measured by the temperature sensor 120 with the estimated value of the temperature change of the distal end portion 10a caused by the heating source 180, and judges whether the difference between the measurement value and the estimated value of the temperature change is within the desired range.

### [Function]

Next, a method of detecting the breakdown of at least one of the heater 110 and the temperature sensor 120 including the fogging prevention operation method according to the present embodiment is described with reference to a flowchart shown in FIG. 8C and FIG. 8D.

The operations in Step 11 to Step 15 and in Step 21 to Step 28 and in Steps 30 and 31 are similar to those in the first embodiment and are therefore not described.

As shown in FIG. 8C, the operation in Step 11, the operation in Step 12, and the operation in Step 21 are sequentially performed.

If yes in Step 21, the operation in Step 22 and the operation in Step 23 are sequentially performed. If no in Step 21, the operation in Step 23 is performed.

If no in Step 23, the operation in Step 24, the operation in Step 13 and the operation in Step 14 are performed. In Step 25, the heater heat quantity calculating section 159 calculates, from an elapsed time of one cycle of the heater driving electric power and the temperature control, a heat quantity of the heater 110 in the cycle, and integrates this heat quantity with the heat quantity of the heater 110 up to the previous cycle of the recording section. The recording section then updates an integration value C1. In Step 25a, the heater heating source heat quantity calculating section 191 then calculates, from an elapsed time of one cycle of the heating source driving electric power and the temperature control, a heat quantity of the heating source 180 in this cycle, and integrates this heat quantity with the heat quantity of the heating source 180 up to the previous cycle of the recording section. The recording section then updates an integration value C2. The operation in Step 15 is then sequentially performed, and the operation returns to Step 11.

If yes in Step 23, the operation in Step 26 and the operation in Step 27 are sequentially performed as shown in FIG. 8D.

After Step 27, the heater temperature estimated value calculating section 161 calculates an estimated value of the temperature change caused by the heater 110 at a desired time based on the heat quantity (integration value C1) of the heater 110 calculated by the heater heat quantity calculating section 159 (Step 28).

The heating source temperature estimated value calculating section 193 calculates an estimated value of the temperature change caused by the heating source 180 at a desired time based on the heat quantity (integration value C2) of the heating source 180 calculated by the heating source heat quantity calculating section 191 (Step 52) .

The correcting section 171 calculates an estimated value of the temperature change of the distal end portion 10a from the estimated value of the temperature change caused by the heater 110 and the estimated value of the temperature change caused by the heating source 180 (Step 53).

The comparing/judging section 163 judges whether the difference between the measurement value of the temperature change of the distal end portion 10a measured by the temperature sensor 120 and the estimated value of the temperature change of the distal end portion 10a is within the desired range (Step 54).

When the comparing/judging section 163 judges that the difference is not within the desired range (Step 54: No), the comparing/judging section 163 judges that at least one of the heater 110 and the temperature sensor 120 has broken down, and outputs the judgment result to the control section 155. In response to this judgment result, the control section 155 deactivates, for example, the heater 110 and the temperature sensor 120. At the same time, the display section 165 displays a warning of deactivation (Step 30) .

When the comparing/judging section 163 judges that the difference is within the desired range (Step 54: Yes), the comparing/judging section 163 judges that the heater 110 and the temperature sensor 120 are normally operating. The operation then proceeds to Step 31.

### [Advantageous effects]

Thus, according to the present embodiment, the estimated value of the temperature change of the distal end portion 10a is calculated based on the calculated heat quantities of the heater 110 and the heating source 180, and these heat quantities are compared with the measurement value of the temperature change of the temperature sensor 120. Thus, according to the present embodiment, it is possible to more high accurately detect whether at least one of the heater 110 and the temperature sensor 120 has broken down or is normally operating.

To improve detection accuracy, the estimated value of the temperature change of the heating source 180 which is always driven as the light guide 20 and the imaging unit 30 may tend to increase up to the saturated state in accordance with the time from the start of the driving of the heating source 180, and may be switched to a constant amount after the saturated state. In the heating source 180 having two states that include the driven state and the deactivated state as the driver element 50, the estimated value of the temperature change of the heating source 180 may be calculated in accordance with the number of times of driving within a desired time.

The heat quantity information may be formed based on the sum of the heat quantity of the heater 110, the heat quantity of the heating source 180, and a heat radiation quantity of the distal end portion 10a. In this case, the heating source heat quantity calculating section calculates the sum of the integration value indicating the heat quantity of the heating source 180 and a negative heat quantity resulting from the heat radiation. The heating source temperature estimated value calculating section 193 has only to then calculate the estimated value of the temperature change of the heating source 180 at a desired time based on the above sum.

The present invention is not completely limited to the embodiments described above, and modifications of components can be made at the stage of carrying out the invention without departing from the scope of the claims. Further, various inventions can be made by properly combining the components disclosed in the embodiments described above.

## Claims

1. An endoscope fogging prevention system (100) comprising:
a heating section (110) which is provided inside a distal end portion (10a) of an endoscope insertion portion (10) and which is adapted to heat the inside of the distal end portion (10a) to prevent the fogging which occurs in an optical member (31, 33) provided inside the distal end portion (10a);
a temperature measurement section (120) which is configured to measure heat quantity information regarding the inside of the distal end portion (10a); and
a control unit (150) which is configured to control the driving of the heating section (110) based on the heat quantity information regarding the inside of the distal end portion (10a) measured by the temperature measurement section (120),
wherein the heat quantity information regarding the inside of the distal end portion (10a) comprises the temperature inside the distal end portion (10a) based on a heat quantity of the heating section (110),
**characterized in that** the control unit (150) is configured to compare a measurement value of a temperature change which is measured by the temperature measurement section (120) over a control time with an estimated value of the temperature change, to judge whether a difference between the measurement value and the estimated value is within a desired range, and to deactivate the heating section (110) when judging that the judgment result is out of the desired range, wherein the estimated value of the temperature change is formed based on an integration value in which a controlled variable for the heating section (110) chosen from electric power, the voltage or the electric current is integrated over said control time.

2. The endoscope fogging prevention system (100) according to claim 1, wherein the heat quantity information is formed based on the sum of the heat quantity of the heating section (110) and the heat quantity of a heating source (180) provided inside the distal end portion.

3. The endoscope fogging prevention system (100) according to claim 2, wherein the heat quantity information is formed based on the sum of the heat quantity of the heater (110) included in the heating section, the heat quantity of the heating source (180), and a heat radiation quantity of the distal end portion (10a).

4. The endoscope fogging prevention system (100) according to claim 3, wherein the heating source (180) has at least one of a light source member (20), an imaging unit (30), and a driving member (50) to drive the imaging unit (30).

5. The endoscope fogging prevention system (100) according to claim 1, further comprising a display section (165) which is configured to display a warning when the control unit (150) judges that the judgment result is out of the desired range.

## Patentansprüche

1. Endoskop-Beschlagschutzsystem (100), aufweisend:
einen Heizabschnitt (110), welcher innerhalb eines distalen Endabschnitts (10a) eines Endoskop-Einführstücks (10) vorgesehen ist und welcher dazu geeignet ist, das Innere des distalen Endabschnitts (10a) zu erwärmen, um das Beschlagen zu vermeiden, welches in einem optischen Bauteil (31, 33) auftritt, das in dem distalen Endabschnitt (10a) vorgesehen ist;
einen Temperatur-Messabschnitt (120), dazu eingerichtet, Wärmemengeninformation bezüglich des Inneren des distalen Endabschnitts (10a) zu messen; und
eine Steuereinheit (150), dazu eingerichtet, den Heizabschnitt (110) basierend auf der Wärmemengeninformation bezüglich des Inneren des distalen Endabschnitts (10a), die von dem Temperatur-Messabschnitt (120) gemessen wird, anzusteuern,
wobei die Wärmemengeninformation bezüglich des Inneren des distalen Endabschnitts (10a) die Temperatur innerhalb des distalen Endabschnitts (10a) basierend auf einer Wärmemenge des Heizabschnitts (110) umfasst,
**dadurch gekennzeichnet, dass** die Steuereinheit (150) dazu eingerichtet ist, einen Messwert einer Temperaturänderung, welche von dem Temperatur-Messabschnitt (120) über eine Regelzeit gemessen wird, mit einem geschätzten Wert der Temperaturänderung zu vergleichen, zu bewerten, ob eine Differenz zwischen dem Messwert und dem geschätzten Wert innerhalb eines gewünschten Bereichs liegt, und den Heizabschnitt (110) zu deaktivieren, wenn bewertet wird, dass das Bewertungsergebnis außerhalb des gewünschten Bereichs liegt, wobei der geschätzte Wert der Temperaturänderung basierend auf einem Integralwert gebildet wird, in welchem eine gesteuerte Variable für den Heizabschnitt (110), die aus der elektrischen Leistung, der Spannung oder dem elektrischen Strom ausgewählt wird, über die besagte Regelzeit integriert wird.

2. Endoskop-Beschlagschutzsystem (100) nach Anspruch 1, wobei die Wärmemengeninformation basierend auf der Summe aus der Wärmemenge von dem Heizabschnitt (110) und der Wärmemenge von einer innerhalb des distalen Endstücks vorgesehenen Wärmequelle (180) gebildet wird.

3. Endoskop-Beschlagschutzsystem (100) nach Anspruch 2, wobei die Wärmemengeninformation basierend auf der Summe aus der Wärmemenge von der Heizung (110), die in dem Heizabschnitt vorhanden ist, der Wärmemenge von der Wärmequelle (180) und der Wärmestrahlungsmenge von dem distalen Endstück (10a) gebildet wird.

4. Endoskop-Beschlagschutzsystem (100) nach Anspruch 3, wobei die Wärmequelle (180) wenigstens eins von einem Lichtquellenbauteil (20), einer Abbildungseinheit (30) und einem Antriebsbauteil (50) zum Antreiben der Abbildungseinheit (30) umfasst.

5. Endoskop-Beschlagschutzsystem (100) nach Anspruch 1, weiter aufweisend einen Anzeigeabschnitt (165), dazu eingerichtet, eine Warnung anzuzeigen, wenn die Steuereinheit (150) bewertet, dass das Bewertungsergebnis außerhalb des gewünschten Bereichs liegt.

## Revendications

1. Système (100) de prévention de la formation de buée sur un endoscope comprenant :
une section de chauffage (110) qui est fournie à l'intérieur d'une partie d'extrémité distale (10a) d'une partie d'insertion d'endoscope (10) et qui est adaptée pour chauffer l'intérieur de la partie d'extrémité distale (10a) pour empêcher l'apparition de buée dans un élément optique (31, 33) fourni à l'intérieur de la partie d'extrémité distale (10a) ;
une section de mesure de température (120) qui est configurée pour mesurer des informations de quantité de chaleur concernant l'intérieur de la partie d'extrémité distale (10a) ; et
une unité de commande (150) qui est configurée pour commander l'entraînement de la section de chauffage (110) sur la base des informations de quantité de chaleur concernant l'intérieur de la partie d'extrémité distale (10a) mesurées par la section de mesure de température (120),
dans lequel les informations de quantité de chaleur concernant l'intérieur de la partie d'extrémité distale (10a) comprennent la température à l'intérieur de la partie d'extrémité distale (10a) sur la base d'une quantité de chaleur de la section de chauffage (110),
**caractérisé en ce que** l'unité de commande (150) est configurée pour comparer une valeur de mesure d'un changement de température qui est mesurée par la section de mesure de température (120) pendant un temps de commande à une valeur estimée du changement de température, pour déterminer si une différence entre la valeur de mesure et la valeur estimée se trouve dans une plage souhaitée, et pour désactiver la section de chauffage (110) quand il est déterminé que le résultat de détermination se trouve hors de la plage souhaitée, dans lequel la valeur estimée du changement de température est formée sur la base d'une valeur d'intégration dans laquelle une variable commandée pour la section de chauffage (110) choisie parmi une puissance électrique, la tension et le courant électrique est intégrée sur ledit temps de commande.

2. Système (100) de prévention de la formation de buée sur un endoscope selon la revendication 1, dans lequel les informations de quantité de chaleur sont formées sur la base de la somme de la quantité de chaleur de la section de chauffage (110) et de la quantité de chaleur d'une source de chauffage (180) fournie à l'intérieur de la partie d'extrémité distale.

3. Système (100) de prévention de la formation de buée sur un endoscope selon la revendication 2, dans lequel les informations de quantité de chaleur sont formées sur la base de la somme de la quantité de chaleur du dispositif chauffant (110) inclut dans la section de chauffage, de la quantité de chaleur de la source de chauffage (180), et d'une quantité de rayonnement de chaleur de la partie d'extrémité distale (10a).

4. Système (100) de prévention de la formation de buée sur un endoscope selon la revendication 3, dans lequel la source de chauffage (180) possède au moins un parmi un élément de source de lumière (20), une unité d'imagerie (30), et un élément d'entraînement (50) pour entraîner l'unité d'imagerie (30).

5. Système (100) de prévention de la formation de buée sur un endoscope selon la revendication 1, comprenant en outre une section d'affichage (165) qui est configurée pour afficher un avertissement quand l'unité de commande (150) détermine que le résultat de détermination se trouve hors de la plage souhaitée.
